# NEW EUROPEAN PATENT SPECIFICATION

(11) **EP 0 644 771 B2**
(45) Date of publication and mention of the opposition decision: **06.12.2006**
(45) Mention of the grant of the patent: 21.08.2002
(21) Application number: 93916466.1
(22) Date of filing: 09.06.1993
(51) Int. Cl.: A61K 38/00, A61K 9/16, A61K 9/50, A61K 9/51

(54) **ERYTHROPOIETIN DRUG DELIVERY SYSTEM**
ENYTHROPOIETIN ENTHALTENDES ARZNEIMITTELABGABESYSTEM
SYSTEME D'APPORT DE MEDICAMENT SOUS FORME D'ERYTHROPOIETINE

(30) Priority: 11.06.1992 US 885307
(43) Date of publication of application: 29.03.1995
(73) Proprietor: Alkermes Controlled Therapeutics, Inc., Cambridge, MA 02139 (US)
(72) Inventor: BERNSTEIN, Howard, Cambridge, MA 02139 (US); MATHIOWITZ, Edith, Brookline, MA 02164 (US); MORREL, Eric, Needham, MA 02194 (US); BRICKNER, Avram, Brookline, MA 02146 (US)
(74) Representative: Thomas, Philip John Duval
(86) International application number: PCT/US1993/005497
(87) International publication number: WO 1993/025221

(56) References cited:
- EP-B1- 0 284 039
- WO-A-89/03678
- WO-A-90/09166
- WO-A-91/12882
- WO-A-92/11844
- WO-A-93/07861
- US-A- 4 806 524
- US-A- 4 822 535
- CHEMICAL ABSTRACTS, vol. 117, no. 6 Columbus, Ohio, US; abstract no. 56020h,

## Description

### Background of the Invention

Erythropoietin (EPO) is a 34 to 38 kda glycoprotein of 165 amino acids, with approximately 40% of its molecular weight provided by carbohydrate, originally isolated from urine, but subsequently produced by recombinant genetic engineering, that is used to increase red blood cell counts. Normal production of human red blood cells requires the secretion of erythropoietin by the kidney, apparently as the mature glycoprotein. In the steady state this hormone circulates in the blood at a concentration of 10 to 18 milliunits (128-230 picograms) per milliliter. The hormone triggers proliferation and differentiation of a population of receptive stem cells in the bone marrow, stimulates hemoglobin synthesis in maturing erythroid cells, and accelerates release of red cells from the marrow into circulation, thereby increasing the red cell mass and ameliorating the hypoxic conditions.

Patients with deficiencies of EPO often suffer severe anemia. EPO is used to treat patients with anemia associated with chronic renal failure, including patients on dialysis (end stage renal disease) and patients not on dialysis, as well as anemia associated with AIDS. EPO is administered as an intravenous bolus three times a week for patients on dialysis and as a subcutaneous injection three times a week for patients not on dialysis. For patients who are receiving EPO on a chronic basis subcutaneously, the frequent injections result in peak and valley plasma levels as well as problems with patient compliance.

It is desirable to find a method and means for oral or parenteral delivery of EPO that is economical, safe and effective.

An oral formulation would be a significant advance in drug administration. An oral formulation would require significant enteric protection from the degradative pathways in the gastrointestinal tract. Microencapsulation of EPO in a suitable polymeric system could protect the incorporated protein from the harsh environment of the intestinal tract. Any microencapsulation process would require mild formulation conditions such as low temperature, physiological pH, and a compatible solvent system for preservation of the biological activity of the EPO.

It is therefore an object of the present invention to provide a method for controlled, delayed release of EPO when administered orally or parenterally.

### summary of the Invention

Biodegradable polymer microspheres, preferably formed of zein, are used for *in vivo* release of erythropoietin (EPO). The microspheres are preferably administered enterally, but can be administered by other routes, for subsequent release. By selecting particular size ranges and the specific polymer to be used to form the microsphere, it is possible to target the microspheres to a cell type such as macrophages, or to effect localized absorption of the microspheres to regions such as the mucosal membranes of the mouth, gastrointestinal tract, or urogenital areas.

Examples demonstrate administration of the EPO in zein and polymer microspheres to dogs and monkeys, with appropriate increases in blood levels and an increase in reticulocyte counts.

### Brief Description of the Drawings

Figure 1 is a graph of the blood serum levels of EPO (mU/ml) overtime (hou rs) in dogs subcutaneously administered EPO in zein microspheres.
Figure 2 is a graph of serum levels of EPO (mU/ml) over time (days) in monkeys enterally administered EPO in zein microspheres.
Figure 3 is a graph of normalized reticulocyte counts over time (days) in monkeys enterally administered EPO in zein microspheres or placebo zein microspheres.
Figure 4 is a graph of serum levels of EPO (mU/ml) overtime (days) in rats subcutaneously administered EPO in PLGA microspheres (squares, rat U10 (5.8 mg), dark squares, rat U11 (2.5 mg)).

### Detailed Description of the Invention

A method of delivery of EPO is described in which polymer microspheres containing the EPO are administered to a human or animal, so that the EPO is released by diffusion from and/or degradation of, the microspheres. The microspheres are formed of biodegradable polymers, most preferably protein polymers such as prolamines or polylactides. A particularly preferred protein is zein. As used herein, polymer refers both to synthetic polymers and proteins. The microspheres are administered orally or by injection, and release the EPO by diffusion and degradation of the microsphere.

The prolamine microspheres have several advantages. The prolamine matrix is a natural, biodegradable substance, which metabolizes in the body to peptides and/or amino acids. The prolamines can be modified, chemically or enzymatically, to endow them with desirable properties, such as a selected degradation rate. The process for making the microspheres from a prolamine solution does not require high temperature heating or cross-linking which could degrade material to be incorporated. Moreover, the microspheres can be designed to be absorbed through the intestinal epithelium into the bloodstream and/or lymphatic system, or targeted to specific organs or phagocytic cells. The microspheres thereby have at least three distinct advantages for controlled delivery: protection of agents that would be attacked and/or degraded by the harsh conditions of the alimentary tract or enzymes in the blood; targeting of a site for release (such as phagocytic cells, mucosal membranes, or the blood); and controlled time and rate of release of agent.

### Incorporation of EPO into microparticles.

EPO containing microspheres are made by incorporating the EPO into a biocompatible polymeric microsphere, wherein the microsphere containing the EPO is characterized by sustained controlled release of the EPO, preferably over a period of at least 24 hours up to a period of one to two months. In the preferred embodiment, the polymer is biodegradable, the microspheres have a diameter of less than one hundred eighty microns, most preferably less than seventy microns, and are suitable for administration by injection subcutaneously or intramuscularly (a size suitable for injection through a 23-gauge needle would be less than 180 µm in diameter), and the microspheres contain from 0.01% by weight up to approximately 20% by weight EPO.

As used herein, "micro" refers to a particle having a diameter of from nanometers to micrometers. Microspheres are solid spherical particles; microparticles are particles of irregular or non-spherical shape. A microsphere may have an outer coating of a different composition than the material originally used to form the microsphere. Unless otherwise noted, the term microspheres can be used to encompass microcapsules, and the term microparticles can be used to encompass microparticles, microspheres, and microcapsules. Microparticulates are specifically referred to when describing irregularly shaped polymer or polymer-drug particles. Microcapsules are spherical shaped polymer devices having a non-polymer core or a core of a different polymer than the outer shell. A "composite microsphere" is a microsphere formed of at least two different materials, either a protein and a polymer or two proteins. A "composite" is an aggregation of microspheres made as described herein, bound by materials known to those skilled in the art for this purpose.

As used herein, "sustained" or "extended" release of the EPO can be continuous or discontinuous, linear or non-linear. This can be accomplished using one or more types of polymer compositions, drug loadings, selections of excipients or degradation enhancers, or other modifications, administered alone, in combination or sequentially to produce the desired effect.

EPO can be incorporated in (1) the polymer matrix forming the microspheres, (2) microparticle(s) surrounded by the polymer which forms the microspheres, (3) a polymer core within a protein microsphere, (4) a polymer coating around a polymer microsphere, (5) mixed in with microspheres aggregated into a larger form, or a combination thereof.

EPO can be incorporated as particulates or by codissolving the EPO with the polymer. Bulk EPO has a specific activity of 120,000 to 180,000 units/mg. It is incorporated into polymer to a concentration of between 50 and 40,000 units/mg polymer. In the preferred embodiment for oral delivery, EPO is dissolved with zein in an aqueous mixture of ethanol. Zein is dissolved in an aqueous organic solution in a concentration range of 3-15% (w/v) with the preferred concentration of 5-8%. Stabilizers can be incorporated by addition of the stabilizers to the EPO solution prior to lyophilization.

### I. Synthetic Polymer Drug Delivery Systems.

### Methods for incorporation of EPO into microspheres.

A variety of techniques are known by which active agents can be incorporated into synthetic polymeric microspheres.

### Spray Drying

In spray drying, the polymer and EPO are mixed together in a solvent for the polymer, then the solvent is evaporated by spraying the solution, leaving polymeric droplets containing the active agent. Spray drying is reviewed in detail by K. Masters in "Spray Drying Handbook" (John Wiley & Sons, New York 1984); and Patrick B. Deasy in "Microencapsulation and Related Drug Processes" (Marcel Dekker, Inc., New York 1984). Spray drying is not preferred since it may result in some loss of activity due to the heat generated in the process as well as in loss of considerable amounts of the material due to sticking of the polymer to the large surface area on the sides of the chamber.

### Solvent Evaporation

Solvent evaporation techniques can be used to form microspheres. These techniques involve dissolving the polymer in an organic solvent which contains either dissolved or dispersed active agent. The polymer/active agent solution is then added to an agitated continuous phase which is usually aqueous. Emulsifiers are included in the aqueous phase to stabilize the oil-in-water emulsion. The organic solvent is then evaporated over a period of several hours or more, thereby depositing the polymer around the core material. Solvent can be removed from the microspheres in a single step, as described in U.S. Patent No. 3,737,337 and U.S. Patent No. 3,523,906, or in U.S. Patent No. 3,691,090 (under reduced pressure), or by the application of heat, as shown in U.S. Patent No. 3,891,570. A two-step technique is described in U.S. Patent No. 4,389,330. Freeze drying has also been used to remove the solvent from microspheres, as reported by Sato, et al, in "Porous Biodegradable Microspheres for Controlled Drug Delivery. I. Assessment of Processing Conditions and Solvent Removal Techniques," Pharmaceutical Research 5, 21-30 (1988).

Solvent evaporation works reasonably well but is not preferred since the amount of incorporated material is usually lower than the theoretical values due to loss of drug to the aqueous phase, as reported by Benita, et al., in "Characterization of Drug Loaded Poly(d,1-lactide) Microspheres," J. Pharm. Sci. 73, 1721-1724 (1984).

### Phase separation

Phase separation techniques can also be used to form microspheres. These techniques involve the formation of a water-in-oil emulsion or oil in water emulsion. The polymer is precipitated from the continuous phase onto the active agent by a change in temperature, pH, ionic strength or the addition of precipitants. For example, U.S. Patent No. 4,675,800, et al., describes the formation of poly(lactic-co-glycolic) acid microspheres containing active proteins. The protein is first dissolved in the aqueous phase of a water-in-oil emulsion or dispersed as a solid in the polymer phase. Polymer is then precipitated around the aqueous droplets or drug particles by addition of a non-solvent for the polymer such as silicone oil. The final product, as with most phase separation techniques, is in the form of a microcapsule. Microcapsules contain a core material surrounded by a polymer membrane capsule. Microcapsules are not the preferred embodiment for delivery of EPo, however, since the release kinetics of active agents from these devices can be difficult to control.

Although these phase separation techniques result in the formation of microspheres containing active agents, active agent is often lost during the solvent extraction process. In addition, as with spray drying, biologically active proteins may be denatured during the process.

### Rapid freezing, solvent extraction

A method for making EPO microspheres having the desired characteristics is described in U.S. Patent No. 5,019,400 to Gombotz, et al.

There are two principal embodiments of the system for making microspheres: a combination liquified gas - frozen non-solvent system and a frozen non-solvent system.

Polymer and agent to be encapsulated in solution are atomized using an ultrasonic device into a liquified gas. The atomized particles freeze when they contact the liquified gas (liquid nitrogen), forming frozen spheres. These sink to the surface of the frozen non-solvent (ethanol). The liquid gas is evaporated and the spheres begin to sink into the non-solvent as the non-solvent thaws. The solvent in the spheres is extracted into the non-solvent to form microspheres containing the agent to be encapsulated. Other non-solvents such as hexane are added to the non-solvent (ethanol) to increase the rate of solvent extraction from certain polymers, where appropriate, for example, when spheres are formed of polylactide-co-glycolide polymers. The liquified gas can be liquid argon (-185.6°C), liquid nitrogen (-195.8°C), liquid oxygen (-182.9°C) or any other gas that results in the immediate freezing of the atomized particles into frozen spheres. Oxygen is not preferred since it is explosive and may cause oxidation of the protein.

Alternatively, a cold non-solvent for the polymer can be substituted for the combination of liquified gas-frozen no-solvent, provided the temperature of the non-solvent is below the freezing temperature of the polymer/active agent solution.

In both embodiments, it is important that the polymer/active agent freeze immediately upon contacting the cold liquid, and then be slowly thawed and the polymer solvent extracted from the microspheres.

The thawing rate is dependent on the choice of solvents and non-solvents. It is important to select a solvent for the polymer having a higher melting point than the non-solvent for the polymer so that the non-solvent melts first, allowing the frozen microspheres to sink into the liquid where they later thaw. If a cold liquid non-solvent system for making the polymeric microspheres is used, the microspheres will sink immediately into the non-solvent. As the solvent in the microsphere thaws, it is extracted into the non-solvent. The solvent for the polymer and the non-solvent for the polymer must be miscible to allow extraction of the solvent from the microspheres. Table 1 shows some polymer/solvent/non-solvent systems that can be used in this process along with their melting points.

**Table 1:**

| **Polymers and Appropriate Solvents and Non-Solvents systems, with Solvent and Non-Solvent Melting Points °C** | | |
|---|---|---|
| POLYMER | SOLVENT | NON-SOLVENT |
| Poly(lactide | Methylene | Ethanol (-114.5) |
| | Chloride (-95.1) | |
| | Chloroform (-63.5) | Methanol (-97.5) |
| Poly(lactide-co-glycolide acid) | Ethyl | Ethanol (-114.5) |
| | Acetate (-83.6) | Ethyl ether (-116.3) |
| | Acetone (-95.4) | |
| | Methylene | Isopentane (-130) |
| | Chloride (-95.1) | |
| Poly(caprolactone) | Methylene | Ethanol (-114.5) |
| | Chloride (-95.1) | |
| Poly (vinyl alcohol) | Water (0) | Acetone (-95.4) |
| Ethylene-vinyl acetate | Methylene | Ethanol (-114.5) |
| | Chloride (-95.0) | |

The polymer/active agent/solvent mixture can be sprayed into the cold liquid, either the liquified gas or the cold non-solvent, using a variety of devices which can be used to form small particles, including sonic nozzles, pressure nozzles, pneumatic nozzles and rotary atomizers.

A wide range of sizes of microspheres can be made by varying the droplet size, for example, by changing the nozzle diameter. If very large spheres are desired, the spheres can be extruded through a syringe directly into the cold liquid. Increasing the inherent viscosity of the polymer solution can also result in an increasing microspheres size. The size of the spheres produced by this process can range from greater than 1000 to 5 microns in diameter. A preferred size range for injectable microspheres is from 30 to 180 microns in diameter. The microspheres made by this technique are spherical in shape.

### Selection of the Synthetic Polymeric Matrix

Almost any type of polymer can be used provided the appropriate solvent and non-solvent are found which have the desired melting points. In general, a polymer solution is prepared containing between 1% polymer and 30% polymer, preferably 5-10% polymer.

The term "bioerodible", or "biodegradable", as used herein refers to materials which are enzymatically or chemically degraded in *vivo* into simpler chemical species.

Polysaccharides are examples of natural polymers. Synthetic polymers that can be used to form the microspheres include bioerodible polymers such as poly(lactide), poly(lactide-co-glycolide), poly(caprolactone), polycarbonates, polyamides, polyanhydrides, polyamino acids, polyortho esters, polyacetals, polycyanoacrylates and degradable polyurethanes, and non-erodible polymers such as polyacrylates, ethylene-vinyl acetate polymers and other acyl substituted cellulose acetates and derivatives thereof, non-erodible polyurethanes, polystyrenes, polyvinyl chloride, polyvinyl fluoride, poly(vinyl imidazole), chlorosulphonated polyolifins, and polyethylene oxide.

The microspheres can be formed of protein, polymer or protein-polymer mixtures. PLA, PGA and PLA/PGA copolymers are particularly useful for forming prolamine composite microspheres. PLA polymers are usually prepared from the cyclic esters of lactic acids. Both L(+) and D(-) forms of lactic acid can be used to prepare the PLA polymers, as well as the optically inactive DL-lactic acid mixture of D(-) and L(+) lactic acids. Methods of preparing polylactides are well documented in the patent literature. The following U.S. Patents, describe in detail suitable polylactides, their properties and their preparation: 1,995,970 to Dorough; 2,703,316 to Schneider; 2,758,987 to Salzberg; 2,951,828 to Zeile; 2,676,945 to Higgins; and 2,683,136; 3,531,561 to Trehu.

PGA is the homopolymer of glycolic acid (hydroxyacetic acid). In the conversion of glycolic acid to poly(glycolic acid), glycolic acid is initially reacted with itself to form the cyclic ester glycolide, which in the presence of heat and a catalyst is converted to a high molecular weight linear-chain polymer. PGA polymers and their properties are described in more detail in Cyanamid Research Develops World's First Synthetic Absorbable Suture", Chemistry and Industry, 905 (1970).

Both the release of the incorporated compound and the bioerosion of the matrix are related to the molecular weights of PLA, PGA or PLA/PGA. The higher molecular weights, weight average molecular weights of 90,000 or higher, result in polymer matrices which retain their structural integrity for longer periods of time; while lower molecular weights, weight average molecular weights of 30,000 or less, result in both slower release and shorter matrix lives.

The release of the EPO from these polymeric systems can occur by two different mechanisms. The drug can be released by diffusion through aqueous filled channels generated in the dosage form by the dissolution of the drug or by voids created by the removal of the polymer solvent during the original microencapsulation. The second mechanism is enhanced release due to the degradation of the polymer. With time the polymer begins to erode and generates increased porosity and microstructure within the device. This creates additional pathways for drug release.

The degradation of the polymers occurs by spontaneous hydrolysis of the ester linkages on the backbone. Thus the rate can be controlled by changing polymer properties influencing water uptake. These include the monomer ratio (lactide to glycolide), the use of L-Lactide as opposed to D/L Lactide, and the polymer molecular weight. These factors determine the hydrophilicity and crystallinity which ultimately govern the rate of water penetration. Hydrophilic excipients such as salts, carbohydrates and surfactants can also be incorporated to increase water penetration into the devices and thus accelerate the erosion of the polymer.

By altering the properties of the polymer and the properties of the dosage form, one can control the contribution of each of these release mechanisms and alter the release rate of EPO. Slowly eroding polymers such as poly L-lactide or high molecular weight poly(lactide-co-glycolide) with low glycolide compositions will cause the release to become diffusion controlled. Increasing the glycolide composition and decreasing the molecular weight enhances both water uptake and the hydrolysis of the polymer and adds an erosion component to the release kinetics.

The release rate can also be controlled by varying the loading of EPO within the microspheres. Increasing the loading will increase the network of interconnecting channels formed upon the dissolution of the drug and enhance the release of drug from the microspheres. The preferred range of EPO loadings is in the range of 40 to 40,000 Units/mg, with the most preferred range being 50 to 20,000 Units/mg.

Polymer hydrolysis is accelerated at acidic or basic pH's and thus the inclusion of acidic or basic excipients can be used to modulate the polymer erosion rate. The excipients can be added as particulates, can be mixed with the incorporated EPo or can be dissolved within the polymer.

Degradation enhancers are based on weight relative to the polymer weight. They can be added to the protein phase, added as a separate phase (i.e., as particulates) or can be codissolved in the polymer phase depending on the compound. In all cases the amount should be between 0.1 and thirty percent (w/w, polymer). Types of degradation enhancers include inorganic acids such as ammonium sulfate and ammonium chloride, organic acids such as citric acid, benzoic acids, heparin, and ascorbic acid, inorganic bases such as sodium carbonate, potassium carbonate, calcium carbonate, zinc carbonate, and zinc hydroxide, and organic bases such as protamine sulfate, spermine, choline, ethanolamine, diethanolamine, and triethanolamine and surfactants such as Tween^{™} and Pluronic^{™}.

Pore forming agents to add microstructure to the matrices (i.e., water soluble compounds such as inorganic salts and sugars). They are added as particulates. The range should be between one and thirty percent (w/w, polymer).

### Additives to alter release rate, degradation rate, stability of EPO

Excipients can be also added to the EPO to maintain its potency depending on the duration of release. Stabilizers include carbohydrates, amino acids, fatty acids, and surfactants and are known to those skilled in the art. In addition, excipients which modify the solubility of EPO such as salts, complexing agents (albumin, protamine) can be used to control the release rate of the protein from the microspheres.

Stabilizers for the EPO are based on ratio to the protein on a weight basis. Examples include carbohydrate such as sucrose, lactose, mannitol, dextran, and heparin, proteins such as albumin and protamine, amino acids such as arginine, glycine, and threonine, surfactants such as Tween^{™} and Pluronic^{™}, salts such as calcium chloride and sodium phosphate, and lipids such as fatty acids, phospholipids, and bile salts.

The ratios are generally 1:10 to 4:1, carbohydrate to protein, amino acids to protein, protein stabilizer to protein, and salts to protein; 1:1000 to 1:20, surfactant to protein; and 1:20 to 4:1, lipids to protein.

### II. Protein Delivery Systems.

### Method of making protein Microparticles.

Using the method described herein, protein microspheres are prepared by a phase separation, solvent removal process. The formation of the microspheres depends upon the differential solubility of proteins in water-miscible organic solvents, salt solutions, or acidic or basic solutions, as compared to their solubility in an immiscible phase, such as a nonpolar organic solvent or an oil. Most proteins are not soluble in oils. Accordingly, protein is dissolved in a first solvent which is a water-miscible organic, organic/aqueous, or binary organic solvent, acid, base or salt solution (the encapsulating phase). The compound to be incorporated, in the form of a suspension, emulsion, solution or particles, is added to the protein solution. This mixture is then contacted with a second liquid phase (the continuous phase) which does not dissolve the proteins and has limited miscibility with the first solvent. The continuous phase is preferably an oil, such as vegetable oil, silicone oil or mineral oil. Vigorous agitation is applied, and the first solvent is removed under conditions sufficient to form microspheres, usually by evaporation or extraction.

Coatings can also be made onto microparticles made of protein or non-protein polymers. To make the coatings, (1) protein is first dissolved in a solvent; (2) the particles or microparticles to be coated are added to the solution; (3) the protein/microparticle mixture is added to a second liquid phase which is immiscible with the first solvent and a non-solvent for the protein coating; (4) the mixture is agitated; and (5) the first solvent is removed (usually by evaporation or extraction) under conditions sufficient to cause the particles or microparticles to be coated with a protein coating.

The process described herein yields protein microspheres having a diameter of between nanometers and micrometers, with an average diameter between 0.01 micron to less than about 100 microns, having incorporated therein a compound to be delivered or released at a desired time and/or site. In the preferred method, the microspheres are stored frozen to enhance the stability of incorporated compounds over extended periods of time.

### Proteins useful for forming the microspheres.

In the preferred embodiments, the proteins are hydrophobic proteins such as prolamines, preferably zein. As used herein, proteins can be a single type of protein, a combination of proteins, or a combination of protein with polymer. Proteins are used to make the microspheres since they are natural, offer a diversity of properties and are degraded *in vivo* into innocuous amino acids or small peptides. Hydrophobic proteins have limited solubility in water and are soluble in organic solvents, aqueous mixtures of organic solvents, and binary mixtures of organic solvents. Examples of other useful proteins besides prolamines are collagen, casein, and keratin.

Prolamines are characterized by having a large number of hydrophobic amino acids, such as glutamine, asparagine and proline. Prolamines are water-insoluble, but are soluble in many organic solvents, particularly alcohols, containing at least one percent (1 %) water, but no more than sixty percent water, or a polar organic solvent.

Prolamines are readily available and inexpensive, for example, as the by-products of grain processing. Representative prolamines include gliadin, kafirin, zein and hordein. A preferred prolamine for use in making microspheres is zein. Both commercially available grades and purified forms of zein can be used. The properties of zein are described in detail by L.C. Swallen in: "Zein - A New Industrial Protein", Ind. and Eng. Chem., 33:394-398 (1941).

### Solvents for the proteins used to form the microspheres.

The protein is dissolved in an appropriate solvent. The protein is "soluble" if more than 0.5% (w/v) of the protein dissolves in the solvent to form a visually transparent solution at room temperature (about 20-25°C). Prolamines are soluble, for example, in alcohols (ethanol), some ketones (e.g., methyl ethyl ketone, acetone) and amide solvents (e.g., acetamide), containing between 5% and 60% water; in extremely high (e.g., pH 10 or greater) or extremely low (pH 2 or less) pH solutions; and in aqueous solutions of from about 1.0 to about 6 N inorganic salts (e.g., NaCl, KBr).

Many binary solvent systems for zein are known, in which the primary components are polyols, especially lower aliphatic alcohols, ketones, or glycols, and the secondary components are water, aromatic hydrocarbons, halogenated hydrocarbons, especially chlorinated hydrocarbons, nitroparaffins, aldehydes and cyclic ethers. Specific examples include mixtures of alcohols and halogenated hydrocarbons and mixtures of alcohols and propylene glycol with ethylene glycol. Binary solvent systems for prolamines such as zein are reported by Manley and Evans, Industrial and Engineering Chemistry 36, 661-665 (1943).

### Suitable materials for the Continuous Phase.

The compound to be incorporated is added to the protein solution. The compound can be in the form of a suspension, solution (in oil, organic solvent or water), emulsion, or particles. The compound/protein mixture is then introduced into a second liquid phase, the continuous phase, which (1) is immiscible or of limited miscibility with the protein solvent and (2) does not dissolve the protein. Solvents are "immiscible" if they will not mix with each other to form a stable homogeneous solution at the operating temperature without mixing. Immiscible phases tend to form separate layers under these conditions. Oils such as mineral oil, silicone oil, or vegetable oil are useful immiscible phases. Others include hexane, heptane, dodecane, and high boiling point petroleum ether.

One or more surfactants can be added to the protein/first solvent mixture or to the continuous phase to reduce the size of the protein microspheres. Suitable surfactants, and methods of use thereof, are known to those skilled in the art.

### Process for forming the Protein Microspheres.

The protein solution was added to the continuous phase, and the first solvent removed, for example, preferably by evaporation, or by solvent extraction, under conditions forming microspheres. Efficient mixing can be achieved by fast mechanical stirring using a homogenizer and/or by using a baffled reactor to prevent laminar flow. If necessary, the mixture can be heated to a temperature of from between 22°C and about 45°C for a period of between about 15 minutes to 45 minutes. If heated, the mixture is first cooled to room temperature, then the microspheres incorporating the compound are washed, separated from the mixture, and dried. If the hydrophilic drug incorporated is unstable in aqueous media, the microspheres can be lyophilized.

In an alternative embodiment used when hydrophilic compounds are to be incorporated into the microspheres other than as particulates, a double emulsion technique is employed. For example, the compound to be incorporated is first dissolved in an aqueous solution. The zein is dissolved in a suitable binary organic mixture with low aqueous miscibility. Many binary organic solvents for zein are known, for example, mixtures of an alcohol, such as methanol, ethanol or isopropanol, with a halogenated hydrocarbon, with the halogenated hydrocarbon as the primary component. The aqueous solution is added to the organic solution of zein and a water in oil emulsion is created. This emulsion is then added to a second organic liquid phase, the continuous phase, which is immiscible or of limited miscibility with the organic solvent for zein, such as an oil, to form a double water in oil emulsion. This solvent is then removed, as described previously, to form microspheres.

### Modification of the microspheres.

The properties of the microspheres can be modified for a given application, for example, by chemically and/or enzymatically altering the starting protein prior to forming the microspheres. Such modifications can produce a protein having enhanced or altered thermal stability, surface reactivity, lipophilicity, molecular weight, charge, shear stability and resistance to proteases.

### Enzymatic modification of the protein.

The functionality, surface properties and molecular weight distribution of the protein can be modified by enzymatic treatment. For example, enzymatic hydrolysis of zein, having a dimer molecularweight of about 38,000 daltons, in 90% ethanol using a protease, such as papain or chymotrypsin, yields polypeptides with a molecular weight of about 1,000 daltons which retain the solubility characteristics of the intact protein, i.e., the polypeptides are still insoluble in water but soluble in 90% ethanol. The degree of hydrolysis can be controlled by varying the amount of enzyme used or the reaction time during which the protein is exposed to the enzyme.

The stability of the protein can be enhanced by crosslinking the protein prior to use in the phase separation process by the addition of an enzyme which catalyzes intra- and/or intermolecular crosslinking of the protein, such as transglutaminase, or protein disulfide isomerase. Transglutaminase and protein disulfide isomerase cause inter- and intramolecular crosslinking of the protein through the amino acids glutamine and cysteine, respectively. Transglutaminase catalyzes an acyl transfer reaction, in which the amide group of the amino acid glutamine is the acyl donor. Other enzymatic processes are known which alter the properties of proteins, before or after formation of the microspheres.

### Chemical modification of the protein.

The properties of the microspheres can also be altered by chemical modification of the proteins used in their preparation, either before or after formation of the microspheres. Such modifications can include treating the proteins with an acid, base or other agent which alters the structure of one or more amino acid side chains, which in turn alters the character of the protein. For example, the high glutamine and asparagine content of prolamines, particularly zein, provides a means for manipulating the charge characteristics of the protein, and therefore the hydrophobicity, by deamidation. The preferred deamidation method involves mild acid-catalyzed deamidation (at a pH of about 1) at elevated temperatures (between 25°C and 65°C) for a period of time sufficient to accomplish the desired level of deamidation. The deamidation process may be followed by measuring the release of ammonia with an ammonia electrode. Deamidation can be terminated by the addition of ammonium carbonate or other base.

Other examples of chemical modification include esterification of the protein with fatty alcohols, or acylation of the protein with fatty anhydrides, which can alter the acid (or base) sensitivity of the protein product. For example, zein or zein peptides can be deamidated as described above, then the deamidated zein reacted with a fatty acid to form the fatty acid ester of the protein. Non-deamidated or deamidated zein peptides can also be reacted with fatty alcohols to form fatty acylated zein or zein peptides. These fatty acid-modified proteins or peptides can then be used as starting material to form the microspheres.

The charge on the protein can also be modified by crosslinking amino acids or polyamino acids to the protein, using glutaraldehyde or carbodiimide.

Proteins can be modified before or after formation of the microspheres. However, an advantage of the phase separation process is that harsh chemical or heat treatment of the protein after formation of the microspheres is not required. Accordingly, when modification of the protein using agents such as glutaraldehyde for crosslinking of the protein is desirable, the protein is treated prior to incorporation of the compound to be delivered and formation of the microspheres.

The same degradation enhancers and stabilizers described with respect to the synthetic polymers can be added to the polymers for the formation of the protein-EPO microspheres.

### III. Formation of protein-polymer microspheres.

Matrices made of either a protein mixture or a protein-polymer mixture, such as prolamine/PLA, prolamine/PGA or prolamine/ PLA-PGA, can be designed with a variety of degradation and diffusion rates. In general, degradation is a function of the protein and polymer composition. Diffusion is a function of the matrix composition, form, and the nature of the incorporated material. Matrices can be synthesized to degrade over periods of time shorter than, equal to or longer than the period of release of incorporated compound. The compound can be released by diffusion, degradation of matrix, or a combination of diffusion through the matrix and release as the matrix degrades.

These composite matrices can take one of several forms: protein microspheres with a polymer coating; polymer microparticles or microcapsules encapsulated by protein; bioactive compounds and protein microspheres encapsulated by polymer; or protein microspheres with or without incorporated bioactive compound and bioactive compound encapsulated by polymer.

### sizes of microspheres.

The microspheres can be produced in a variety of sizes, ranging from nanometer-sized microspheres up to an average size of about 100 microns. Microspheres having an average particle size of from about 50 to 100 nm to about 20 microns are more preferred for enteral administration or targeting to cells. Microspheres having an average particle size of from about 100 nm to about 5 microns are particularly preferred for use in drug delivery because microspheres in this size range may be absorbed into the bloodstream and/or lymphatic system or phagocytized.

The size and other characteristics of the microspheres can be determined using scanning electron microscopy (SEM), light scattering and differential scanning calorimetry (DSC).

### Preparation of protein coatings.

Protein coatings are made using a variation of the method to make microspheres. Particles (including particles of non-uniform shape, microspheres and microcapsules) to be coated can be made from any polymeric substance, usually non-protein substances or modified proteins, or material to be released. To form the coating, the protein is dissolved, the particles to be coated added to the protein solution, the protein/microparticle mixture added to the continuous phase, the mixture agitated and the solvent removed, preferably by evaporation, or by solvent extraction, under conditions causing the particles to be coated with a protein coating.

### IV. Methods for administration of compounds incorporated into microspheres or implants formed from microspheres.

The microspheres can be administered topically, locally or systemically by parenteral administration or enteral administration. The preferred route is oral for microspheres made from protein polymers.

### Enteral Administration.

Microspheres containing EPO are preferably administered orally. These microspheres, depending on the chemical nature and size, will either be absorbed to, or passed through, the epithelial lining of the gastrointestinal tract into the bloodstream or lymphatic system. The EPO is released from the microspheres by diffusion, degradation, or a combination of degradation and diffusion, in the blood stream, lymphatic system, epithelium, or at the surface of the epithelium.

### Parenteral Administration.

Microspheres of less than 180 microns readily pass through a needle for intravenous administration. Suitable pharmaceutical carriers, for example, a phosphate buffered saline, are known and commercially available.

### Subcutaneous, Intramuscular and Intraperitoneal Administration.

Microspheres produced as described above are small enough to be injected through a standard gauge needle under the skin or into the peritoneum for subsequent release of incorporated drug. Adhesion of the microspheres to the peritoneum aids in localizing release of the incorporated drug. Microspheres can also be implanted or injected intramuscularly for immunization or other purposes where slower release into the bloodstream is desirable. Carriers such as phosphate buffer saline, or an adjuvant such as an oil, can be used as a carrier for the microspheres. Pharmaceutically acceptable carriers are known to those skilled in the art.

The methods are further described with reference to specific embodiments demonstrating incorporation and release of biologically active EPO.

### Example 1: Preparation of Prolamine microspheres containing EPO.

A solution of EPO (1.0 mg/ml EPO in 400 mM sodium chloride, 8.0 mM sodium phosphate and 1.5 mM potassium phosphate) was quick frozen in liquid nitrogen and lyophilized for 48 hours. The lyophilized powder had a specific activity of 60,000 units/mg total solids.

Lyophilized EPO (2.0 mg) was dissolved in 200 µl water. 200 mg zein (Type F5000, Freeman Industries, Tucahoe, NY) was dissolved in 3.8 ml of an aqueous ethanol mixture (200 µl water and 3.6 ml 100% ethanol). The zein solution was then added to the EPO solution and gently mixed. The zein EPO solution was then emulsified for 20 seconds with 120 ml corn oil using a Virtishear homogenizer and an ultrafine generator. This emulsion was then added to 250 ml corn oil preheated to 60°C and mixed with an overhead mixer at 1800 rpm. The resulting mixture had a temperature of 50°C. The temperature was maintained in the range 45 to 50°C with a heating block for twenty minutes, after which the microspheres were cooled to room temperature. The resulting microspheres were washed with petroleum ether to remove the corn oil and filtered. The microspheres were dried overnight under vacuum at room temperature.

### Example 2: In vivo: release of EPO from Prolamine microspheres administered subcutaneously to dogs.

The zein microspheres formed in example 1 were tested in a subcutaneous administration model in beagle dogs. The dogs (N=2) were injected subcutaneously with a single dose of zein microspheres (30 mg) with 1% EPO solids. Blood samples for the measurement of EPO were taken for 96 hours post injection. Serum was prepared and assayed for EPO by radioimmunoassay (Incstar, Stillwater, MN).

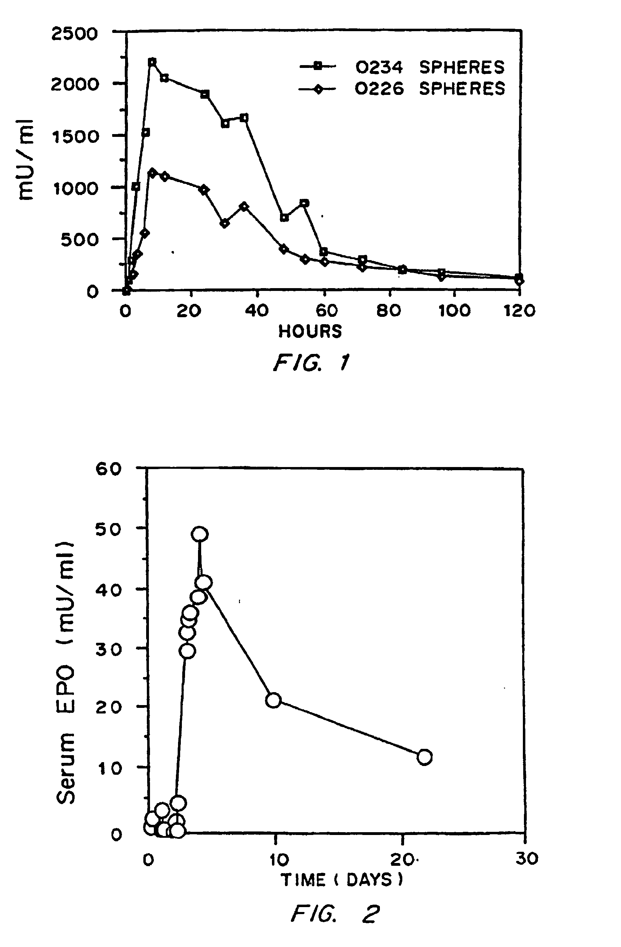

The increase in serum EPO for each of the animals in shown in Figure 1. The plasma levels peak at ten hours and return to baseline by 120 hours after administration.

### Example 3: In vivo release of EPO from Prolamine microspheres administered orally to monkeys.

The zein EPO microspheres formed in Example 1 were tested in an enteral administration model in cynomolgus monkeys. the animals (N=6) were fed by gavage a single dose of 200 mg zein microspheres with 1% EPO solids on days one, two and three of the study. One hour before dosing, each monkey received a single intramuscular injection of 20 mg of cimetidine to raise gastric pH. The microspheres were resuspended in 8 ml of a phosphate buffered resuspension medium. The gavage tube was subsequently flushed with 4 ml of resuspension medium.

Each animal also underwent a control experiment in which a single dose of blank microspheres without any active drug was fed by gavage on days one, two and three of the study. One hour before dosing each monkey received a single intramuscular injection of 20 mg of cimetidine to raise gastric pH. Each animal was rested for two months between the placebo and active experiments.

Blood samples for measurement of EPO were taken prior to each gavage and then two, four, and six hours after dosing. Blood samples were also taken on the day after the last dose, on the tenth day after dosing, and on the twenty-second day after dosing. Blood samples were taken at the same sampling times for both studies. Serum was prepared and was assayed for EPO using a radioimmunoassay (INCSTAR, Stillwater, MN).

Samples for reticulocyte counts were collected on study day 1, prior to microsphere administration, and on days 5, 10, 20 after dosing.

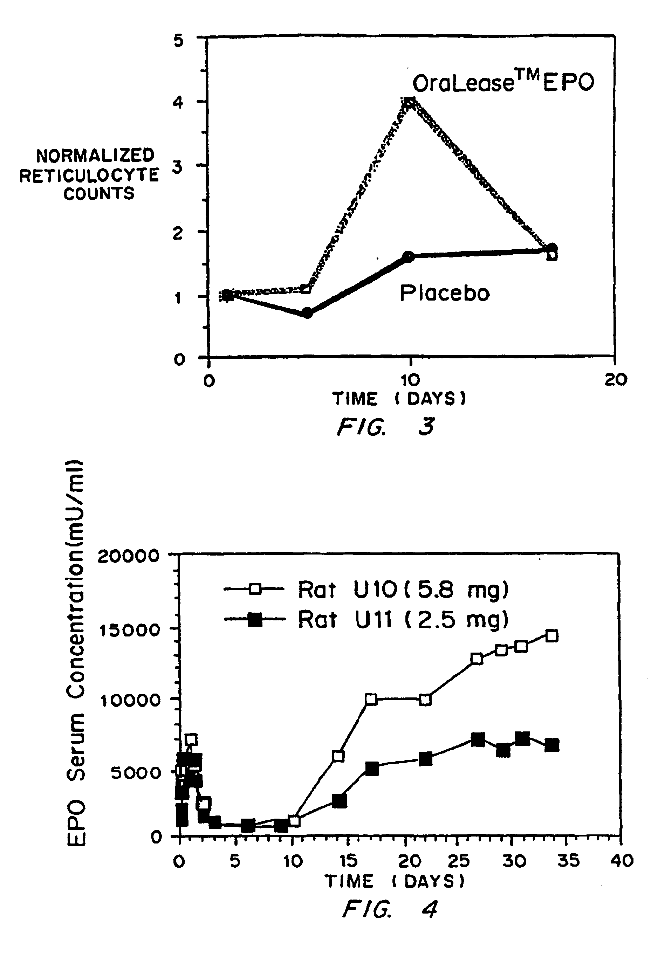

The increase in mean serum EPO level is shown in Figure 2. The EPO level increases to a maximum of 50 mU/ml after day 5, and returns to baseline after 22 days. The normalized reticulocyte counts are shown in Figure 3. The count peaks at day 10 at 3.5 times the baseline reticulocyte count. The increase in reticulocyte counts demonstrates that the absorbed EPO has biological activity.

### Example 4: Preparation of Polymer microspheres containing EPO.

0.25 grams of poly D/L lactide (Boehringer Ingelheim, Resomer R104) and 0.25 grams of poly D/L lactide co-glycolide (Birmingham Polymer Inc., Lactel 50:50) was dissolved in 5.0 ml of methylene chloride. To this polymer solution was added 15 mg of desalted lyophilized EPO powder containing 1% w/w Pluronics^{™} F68 (BASF). The lyophilized EPO had particle sizes in the range of one to six microns. The suspension was placed in a ten ml gas tight syringe. A 200 ml amount of 100% ethanol was added to a round polypropylene container (17 cm diameter, 8 cm deep). This solution was frozen in liquid nitrogen and covered with 500 ml of liquid nitrogen. The polymer protein mixture was pumped from the syringe via a syringe pump at 2 ml/min, into an ultrasonic nozzle (Mode, Sonics and Material, Danbury, CT) that was placed above the container of liquid nitrogen and frozen ethanol. The nozzle atomized the suspension into droplets which froze upon contact with the liquid nitrogen and formed microspheres which sank onto the frozen ethanol.

The container was placed at -80°C where the liquid nitrogen evaporated and the ethanol melted with time. As the ethanol thaws, the microspheres settle into the liquid where the methylene chloride is extracted. After 24 hours, an additional 200 ml of hexane prechilled to -80°C was added to the container. After three days, the slurry of microspheres and ethanol was filtered using a one micron Durapore^{™} membrane (Millipore, Bedford, MA). The filtered microspheres were then lyophilized.

The microspheres formed in example 4 were tested in a subcutaneous administration model in Sprague Dawley rats. The rats (N=2) were injected subcutaneously with a single dose of microspheres (2.5 mg or 5.8 mg). Blood samples for the measurement of EPO were taken for several weeks post injection. Serum was prepared and was assayed by EPO by radioimmunoassay (Incstar, Stillwater, MN).

The increase in serum EPO (mU/ml) for each of the animals is shown in Figure 4. The plasma profile is biphasic with the first phase lasting for three days which is followed by an induction period of one week after which the levels remain above baseline for at least three weeks.

## Claims (Claims for the following Contracting State(s): AT, BE, CH, DE, DK, ES, FR, GB, GR, IT, LI, LU, MC, NL, SE)

1. A method for making a sustained release composition for biologically active erythropoietin for administration to a patient comprising incorporating the erythropoietin into a biodegradable polymeric microsphere so that said erythropoietin is incorporated into the polymer of said microsphere, wherein the erythropoietin is in a concentration of between 0.01 to 20% by weight, and in combination with a stabilizing agent selected from the group consisting of amino acids, surfactants, salts, and lipids, wherein the ratio of stabilizing agent to erythropoietin protein is between 1:10 and 4:1 for amino acids to protein and salt to protein, between 1:1000 and 1:20 for surfactant to protein, and between 1:20 and 4:1 for lipids to protein.

2. The method of Claim 1 wherein the microsphere is a protein microsphere produced by
(a) contacting a protein solution containing at least one type of protein with a second liquid, which is of limited miscibility with the protein solvent and does not dissolve the protein, to form a protein-non-solvent mixture;
(b) agitating the protein-non-solvent mixture to form a dispersion of the protein solution in the second liquid; and
(c) removing the protein solvent to form protein microsphere;
wherein the protein microsphere has a diameter between 50 nm and 100 microns and the microsphere is not formed by amide linkages or heat denaturation of the protein.

3. The method of Claim 2 wherein the protein is hydrophobic protein.

4. The method of Claim 3 wherein the hydrophobic protein is selected from the group consisting of prolamine, collagen, casein, and keratin.

5. The method of Claim 1 wherein the microsphere is formed of a synthetic polymer selected from the group consisting of poly(lactide), poly(lactide-co-glycolide), poly(caprolactone), polycarbonates, polyamides, polyanhydrides, polyamino acids, polyortho esters, polyacetals, polycyanoacrylates, polyurethanes, polyacrylates, ethylene-vinyl acetate polymers, polystyrenes, polyvinyl chloride, polyvinyl fluoride, poly(vinyl imidazole), chlorosulphonated polyolefins, polyethylene oxide, polyphosphazene, polyhydroxybutyrate, blends and copolyers thereof.

6. The method of Claim 1 wherein the microsphere is formed of a non-protein polymer in combination with a protein.

7. The method of Claim 1 wherein the microspheres are in a pharmaceutical carrier for enteral administration to a patient.

8. The method of Claim 1 wherein the microspheres are in a pharmaceutical carrier for parenteral administration to a patient.

9. A sustained release composition for biologically active erythropoietin comprising erythropoietin incorporated into the polymer of a biodegradable polymeric microsphere, wherein the erythropoietin is in a concentration of between 0.01 to 20% by weight, containing between 50 and 40,000 Units/mg, and in combination with a stabilizing agent selected from the group consisting of amino acids, surfactants, salts and lipids, wherein the ratio of stabilizing agent to erythropoietin protein is between 1:10 and 4:1 for amino acids to protein, and salt to protein, between 1:1000 and 1:20 for surfactant to protein, and between 1:20 and 4:1 for lipids to protein.

10. The composition of Claim 9 wherein the biodegradable polymeric microsphere is as defined in any one of Claims 3 to 6.

11. The composition of Claims 9 or 10 wherein the microspheres are in a pharmaceutically acceptable carrier.

12. The composition of Claim 9 further comprising excipients modulating polymer erosion rate selected from the group consisting of inorganic acids, inorganic bases, organic bases, organic acids, salts, complexing agents, carbohydrates and surfactants.

13. The composition of Claim 12 wherein the excipient modulating polymer erosion rate is a pore forming agent such as a salt or sugar and is added to the polymer in particulate form in a concentration of between one and thirty percent (w/w, polymer).

14. The composition of Claim 9 further comprising excipients modifying the solubility of EPO selected from the group consisting of salts, inorganic acids, organic acids, inorganic bases, organic bases, and surfactants and being present in a concentration of between 0.1 and thirty percent (w/w, polymer).

15. A composition obtainable by the method of any one of Claims 1 to 9.

16. A sustained release composition for biologically active erythropoietin comprising erythropoietin incorporated into the polymer of a biodegradable polymeric microsphere, wherein the erythropoietin is in a concentration of between 0.01 to 20% by weight, ammonium sulphate, a metal carbonate and a stabilizing agent selected from the group consisting of amino acids, surfactants, salts, and lipids, wherein the ratio of stabilizing agent to erythropoietin protein is between 1:10 and 4:1 for amino acids to protein and salt to protein, between 1:1000 and 1:20 for surfactant to protein, and between 1:20 and 4:1 for lipids to protein.

## Claims (Claims for the following Contracting State(s): IE, PT)

1. A method for making a sustained release composition for biologically active erythropoietin for administration to a patient comprising incorporating the erythropoietin into a biodegradable polymeric microsphere so that said erythropoietin is incorporated into the polymer of said microsphere, wherein the erythropoietin is in a concentration of between 0.01 to 20% by weight, and in combination with a stabilizing agent selected from the group consisting of carbohydrates, proteins, amino acids, surfactants, salts, and lipids, wherein the ratio of stabilizing agent to erythropoietin protein is between 1:10 and 4:1 for carbohydrate to protein, amino acids to protein, protein stabilizer to protein, and salt to protein, between 1:1000 and 1:20 for surfactant to protein, and between 1:20 and 4:1 for lipids to protein.

2. The method of Claim 1 wherein the microsphere is a protein microsphere produced by
(a) contacting a protein solution containing at least one type of protein with a second liquid, which is of limited miscibility with the protein solvent and does not dissolve the protein, to form a protein-non-solvent mixture;
(b) agitating the protein-non-solvent mixture to form a dispersion of the protein solution in the second liquid; and
(c) removing the protein solvent to form protein microsphere;
wherein the protein microsphere has a diameter between 50 nm and 100 microns and the microsphere is not formed by amide linkages or heat denaturation of the protein.

3. The method of Claim 2 wherein the protein is hydrophobic protein.

4. The method of Claim 3 wherein the hydrophobic protein is selected from the group consisting of prolamine, collagen, casein, and keratin.

5. The method of Claim 1 wherein the microsphere is formed of a synthetic polymer selected from the group consisting of poly(lactide), poly(lactide-co-glycolide), poly(caprolactone), polycarbonates, polyamides, polyanhydrides, polyamino acids, polyortho esters, polyacetals, polycyanoacrylates, polyurethanes, polyacrylates, ethylene-vinyl acetate polymers, polystyrenes, polyvinyl chloride, polyvinyl fluoride, poly(vinyl imidazole), chlorosulphonated polyolefins, polyethylene oxide, polyphosphazene, polyhydroxybutyrate, blends and copolyers thereof.

6. The method of Claim 1 wherein the microsphere is formed of a non-protein polymer in combination with a protein.

7. The method of Claim 1 wherein the microspheres are in a pharmaceutical carrier for enteral administration to a patient.

8. The method of Claim 1 wherein the microspheres are in a pharmaceutical carrier for parenteral administration to a patient.

9. A sustained release composition for biologically active erythropoietin comprising erythropoietin incorporated into the polymer of a biodegradable polymeric microsphere, wherein the erythropoietin is in a concentration of between 0.01 to 20% by weight, containing between 50 and 40,000 Units/mg, and in combination with a stabilizing agent selected from the group consisting of carbohydrates, proteins, amino acids, surfactants, salts, and lipids, wherein the ratio of stabilizing agent to erythropoietin protein is between 1:10 and 4:1 for carbohydrate to protein, amino acids to protein, protein stabilizer to protein, and salt to protein, between 1:1000 and 1:20 for surfactant to protein, and between 1:20 and 4:1 for lipids to protein.

10. The composition of Claim 9 wherein the biodegradable polymeric microsphere is as defined in any one of Claims 3 to 6.

11. The composition of Claims 9 or 10 wherein the microspheres are in a pharmaceutically acceptable carrier.

## Patentansprüche (Patentansprüche für folgende(n) Vertragsstaat(en): AT, BE, CH, DE, DK, ES, FR, GB, GR, IT, LI, LU, MC, NL, SE)

1. Verfahren zur Herstellung einer Zusammensetzung mit verzögerter Freisetzung für biologisch aktives Erythropoietin zur Verabreichung an einen Patienten, umfassend das Einarbeiten des Erythropoietins in ein biologisch abbaubares Polymermikrokügelchen, so dass das Erythropoietin in das Polymer des Mikrokügelchens eingearbeitet ist, wobei das Erythropoietin in einer Konzentration von zwischen 0,01 und 20 Gew.-% und in Kombination mit einem Stabilisierungsmittel, das aus Aminosäuren, Netzmitteln, Salzen und Lipiden ausgewählt ist, wobei das Verhältnis von Stabilisierungsmittel zu Erythropoietinprotein zwischen 1:10 und 4:1 für Aminosäuren gegenüber Protein und Salz gegenüber Protein, zwischen 1:1000 und 1:20 für ein Netzmittel gegenüber Protein und zwischen 1:20 und 4:1 für Lipide gegenüber Protein beträgt, vorhanden ist.

2. Verfahren gemäß Anspruch 1, wobei das Mikrokügelchen ein Proteinmikrokügelchen ist, das hergestellt wird durch
a) In-Kontakt-Bringen einer Proteinlösung, die mindestens eine Art Protein enthält, mit einer zweiten Flüssigkeit, die von begrenzter Mischbarkeit mit dem Proteinlösemittel ist und das Protein nicht löst, zur Bildung eines Protein-Nichtlösemittel-Gemischs;
b) Rühren des Protein-Nichtlösemittel-Gemischs zur Bildung einer Dispersion der Proteinlösung in der zweiten Flüssigkeit ; und
c) Entfernen des Proteinlösemittels zur Bildung von Proteinmikrokügelchen,
wobei das Proteinmikrokügelchen einen Durchmesser zwischen 50 nm und 100 *µ*m aufweist und das Mikrokügelchen nicht durch Amidbindungen oder Hitzedenaturierung des Proteins gebildet wird.

3. Verfahren nach Anspruch 2, wobei das Protein ein hydrophobes Protein ist.

4. Verfahren nach Anspruch 3, wobei das hydrophobe Protein aus der aus Prolamin, Collagen, Casein und Keratin bestehenden Gruppe ausgewählt ist.

5. Verfahren nach Anspruch 1, wobei das Mikrokügelchen aus einem synthetischen Polymer, das aus der Gruppe aus Poly(lactid), Poly(lactid-co-glycolid), Poly(caprolacton), Polycarbonaten, Polyamiden, Polyanhydriden, Polyaminosäuren, Polyorthoestern, Polyacetalen, Polycyanoacrylaten, Polyurethanen, Polyacrylaten, Ethylen-Vinylacetatpolymeren, Polystyrolen, Polyvinylchlorid, Polyvinylfluorid, Poly(vinylimidazol), chlorsulfonierten Polyolefinen, Polyethylenoxid, Polyphosphazen, Polyhydroxybutyrat, Gemischen und Copolymeren derselben ausgewählt ist, gebildet ist.

6. Verfahren nach Anspruch 1, wobei das Mikrokügelchen aus einem Nichtproteinpolymer in Kombination mit einem Protein gebildet ist.

7. Verfahren nach Anspruch 1, wobei die Mikrokügelchen in einem pharmazeutischen Träger zur enteralen Verabreichung an einen Patienten sind.

8. Verfahren nach Anspruch 1, wobei die Mikrokügelchen in einem pharmazeutischen Träger zur parenteralen Verabreichung an einen Patienten sind.

9. Zusammensetzung mit verzögerter Freisetzung für biologisch aktives Erythropoietin, die in das Polymer eines biologisch abbaubaren Polymermikrokügelchens eingearbeitetes Erythropoietin umfasst, wobei das Erythropoietin in einer Konzentration von zwischen 0,01 und 20 Gew.-%, wobei zwischen 50 und 40000 Einheiten/mg enthalten sind, und in Kombination mit einem Stabilisierungsmittel, das aus Aminosäuren, Netzmitteln, Salzen und Lipiden ausgewählt ist, vorhanden ist, wobei das Verhältnis von Stabilisierungsmittel zu Erythropoietinprotein zwischen 1:10 und 4:1 für Aminosäuren gegenüber Protein und Salz gegenüber Protein, zwischen 1:1000 und 1:20 für Netzmittel gegenüber Protein und zwischen 1:20 und 4:1 für Lipide gegenüber Protein beträgt.

10. Zusammensetzung nach Anspruch 9, wobei das biologisch abbaubare Polymermikrokügelchen wie in einem der Ansrpüche 3 bis 6 definiert ist.

11. Zusammensetzung nach Anspruch 9 oder 10, wobei die Mikrokügelchen in einem pharmazeutisch akzeptablen Träger sind.

12. Zusammensetzung nach Anspruch 9, die ferner Streckmittel, die die Polymererosionsrate modulieren, die aus der aus anorganischen Säuren, anorganischen Basen, organischen Basen, organischen Säuren, Salzen, Komplexbildnern, Kohlehydraten und Netzmitteln bestehenden Gruppe ausgewählt sind, umfasst.

13. Zusammensetzung nach Anspruch 12, wobei das Streckmittel, das die Polymererosionsrate moduliert, ein Porenbildungsmittel, beispielsweise ein Salz oder Zucker, ist und dem Polymer in Teilchenform in einer Konzentration von zwischen und 1 und 30 % (Gew/Gew, Polymer) zugesetzt wird.

14. Zusammensetzung nach Anspruch 9, die ferner Streckmittel umfasst, die die Löslichkeit von EPO modifizieren, die aus der aus Salzen, anorganischen Säuren, organischen Säuren, anorganischen Basen, organischen Basen und Netzmitteln bestehenden Gruppe ausgewählt sind und in einer Konzentration von zwischen 0,1 und 30 % (Gew/Gew, Polymer) vorhanden sind.

15. Zusammensetzung, die durch das Verfahren nach einem der Ansprüche 1 bis 9 erhalten werden kann.

16. Zusammensetzung mit verzögerter Freisetzung für biologisch aktives Erythropoietin, die in das Polymer eines biologisch abbaubaren Polymermikrokügelchens eingearbeitetes Erythropoietin, wobei das Erythropoietin in einer Konzentration von zwischen 0,01 bis 20 Gew.-% vorliegt, Ammoniumsulfat, ein Metallcarbonat und ein Stabilisierungsmittel, das aus der Gruppe von Aminosäuren, Netzmiteln, Salzen und Lipiden ausgewählt ist, umfasst, wobei das Verhältnis von Stabilisierungsmittel zu Erythropoietinprotein zwischen 1:10 und 4:1 für Aminosäuren gegenüber Protein und für Salz gegenüber Protein, zwischen 1:1000 und 1:20 für Netzmittel gegenüber Protein und zwischen 1:20 und 4:1 für Lipide gegenüber Protein liegt.

## Patentansprüche (Patentansprüche für folgende(n) Vertragsstaat(en): IE, PT)

1. Verfahren zur Herstellung einer Zusammensetzung mit verzögerter Freisetzung für biologisch aktives Erythropoietin zur Verabreichung an einen Patienten, umfassend das Einarbeiten des Erythropoietins in ein biologisch abbaubares Polymermikrokügelchen, so dass das Erythropoietin in das Polymer des Mikrokügelchens eingearbeitet ist, wobei das Erythropoietin in einer Konzentration von zwischen 0,01 und 20 Gew.-% und in Kombination mit einem Stabilisierungsmittel, das aus Kohlehydraten, Proteinen, Aminosäuren, Netzmitteln, Salzen und Lipiden ausgewählt ist, wobei das Verhältnis von Stabilisierungsmittel zu Erythropoietinprotein zwischen 1:10 und 4:1 für Kohlehydrat gegenüber Protein, Aminosäuren gegenüber Protein, Proteinstabilisator gegenüber Protein und Salz gegenüber Protein, zwischen 1:1000 und 1:20 für ein Netzmittel gegenüber Protein und zwischen 1:20 und 4:1 für Lipide gegenüber Protein beträgt, vorhanden ist.

2. Verfahren gemäß Anspruch 1, wobei das Mikrokügelchen ein Proteinmikrokügelchen ist, das hergestellt wird durch
a) In-Kontakt-Bringen einer Proteinlösung, die mindestens eine Art Protein enthält, mit einer zweiten Flüssigkeit, die von begrenzter Mischbarkeit mit dem Proteinlösemittel ist und das Protein nicht löst, zur Bildung eines Protein-Nichtlösemittel-Gemischs;
b) Rühren des Protein-Nichtlösemittel-Gemischs zur Bildung einer Dispersion der Proteinlösung in der zweiten Flüssigkeit; und
c) Entfernen des Proteinlösemittels zur Bildung von Proteinmikrokügelchen,
wobei das Proteinmikrokügelchen einen Durchmesser zwischen 50 nm und 100 *µ*m aufweist und das Mikrokügelchen nicht durch Amidbindungen oder Hitzedenaturierung des Proteins gebildet wird.

3. Verfahren nach Anspruch 2, wobei das Protein ein hydrophobes Protein ist.

4. Verfahren nach Anspruch 3, wobei das hydrophobe Protein aus der aus Prolamin, Collagen, Casein und Keratin bestehenden Gruppe ausgewählt ist.

5. Verfahren nach Anspruch 1, wobei das Mikrokügelchen aus einem synthetischen Polymer, das aus der Gruppe aus Poly(lactid), Poly(lactid-co-glycolid), Poly(caprolacton), Polycarbonaten, Polyamiden, Polyanhydriden, Polyaminosäuren, Polyorthoestern, Polyacetalen, Polycyanoacrylaten, Polyurethanen, Polyacrylaten, Ethylen-Vinylacetatpolymeren, Polystyrolen, Polyvinylchlorid, Polyvinylfluorid, Poly(vinylimidazol), chlorsulfonierten Polyolefinen, Polyethylenoxid, Polyphosphazen, Polyhydroxybutyrat, Gemischen und Copolymeren derselben ausgewählt ist, gebildet ist.

6. Verfahren nach Anspruch 1, wobei das Mikrokügelchen aus einem Nichtproteinpolymer in Kombination mit einem Protein gebildet ist.

7. Verfahren nach Anspruch 1, wobei die Mikrokügelchen in einem pharmazeutischen Träger zur enteralen Verabreichung an einen Patienten sind.

8. Verfahren nach Anspruch 1, wobei die Mikrokügelchen in einem pharmazeutischen Träger zur parenteralen Verabreichung an einen Patienten sind.

9. Zusammensetzung mit verzögerter Freisetzung für biologisch aktives Erythropoietin, die in das Polymer eines biologisch abbaubaren Polymermikrokügelchens eingearbeitetes Erythropoietin umfasst, wobei das Erythropoietin in einer Konzentration von zwischen 0,01 und 20 Gew.-%, wobei zwischen 50 und 40000 Einheiten/mg enthalten sind, und in Kombination mit einem Stabilisierungsmittel, das aus Kohlehydraten, Proteinen, Aminosäuren, Netzmitteln, Salzen und Lipiden ausgewählt ist, vorhanden ist, wobei das Verhältnis von Stabilisierungsmittel zu Erythropoietinprotein zwischen 1:10 und 4:1 für Kohlehydrat gegenüber Protein, Aminosäuren gegenüber Protein, Proteinstabilisator gegenüber Protein und Salz gegenüber Protein, zwischen 1:1000 und 1:20 für Netzmittel gegenüber Protein und zwischen 1:20 und 4:1 für Lipide gegenüber Protein beträgt.

10. Zusammensetzung nach Anspruch 9, wobei das biologisch abbaubare Polymermikrokügelchen wie in einem der Ansprüche 3 bis 6 definiert ist.

11. Zusammensetzung nach Anspruch 9 oder 10, wobei die Mikrokügelchen in einem pharmazeutisch akzeptablen Träger sind.

## Revendications (Revendications pour l'(les) Etat(s) contractant(s) suivant(s): AT, BE, CH, DE, DK, ES, FR, GB, GR, IT, LI, LU, MC, NL, SE)

1. Procédé de fabrication d'une composition à libération prolongée pour l'érythropoïétine biologiquement active pour administration à un patient, comprenant l'incorporation de l'érythropoïétine dans une microsphère polymère biodégradable de manière à ce que ladite érythropoïétine soit incorporée dans le polymère de ladite microsphère, dans lequel l'érythropoïétine est dans une concentration située entre 0,01 et 20 % en poids, et en combinaison avec un agent stabilisant choisi parmi le groupe constitué des acides aminés, des tensioactifs, des sels et des lipides, dans lequel le rapport de l'agent stabilisant sur la protéine de l'érythropoïétine est situé entre 1 : 10 et 4 : 1 pour les acides aminés sur la protéine et le sel sur la protéine, entre 1 : 1000 et 1 : 20 pour le tensioactif sur la protéine et entre 1 : 20 et 4 : 1 pour les lipides sur la protéine.

2. Procédé de la revendication 1, dans lequel la microsphère est une microsphère de protéine produite en
a) mettant en contact une solution de protéine contenant au moins un type de protéine avec un deuxième liquide, qui est d'une miscibilité limitée avec le solvant de protéine et ne dissout pas la protéine pour former un mélange de protéine et de non solvant ;
b) agitant le mélange de protéine et de non solvant pour former une dispersion de la solution de protéine dans le deuxième liquide ; et
c) éliminant le solvant de protéine pour former une microsphère de protéine,
dans lequel la microsphère de protéine a un diamètre situé entre 50 nm et 100 microns et la microsphère n'est pas formée par des liaisons amide ou une dénaturation thermique de la protéine.

3. Procédé de la revendication 2, dans lequel la protéine est une protéine hydrophobe.

4. Procédé de la revendication 3, dans lequel la protéine hydrophobe est choisie parmi le groupe constitué de la prolamine, du collagène, de la caséine et de la kératine.

5. Procédé de la revendication 1, dans lequel la microsphère est formée d'un polymère synthétique choisi parmi le groupe constitué des poly(lactides), poly(lactides-co-glycolides), poly(caprolactones), polycarbonates, polyamides, polyanhydrides, polyaminoacides, polyortho-esters, polyacétals, polycyanoacrylates, polyuréthannes, polyacrylates, polymères éthylène - acétate de vinyle, polystyrènes, poly(chlorures de vinyle), poly(fluorures de vinyle), poly(vinyl imidazoles), polyoléfines chlorosulfonées, poly(oxydes d'éthylène), polyphosphazènes, polyhydroxybutyrates, de leurs mélanges et copolymères.

6. Procédé de la revendication 1, dans lequel la microsphère est formée d'un polymère non protéine en combinaison avec une protéine.

7. Procédé de la revendication 1, dans lequel les microsphères sont dans un support pharmaceutique pour l'administration entérale à un patient.

8. Procédé de la revendication 1, dans lequel les microsphères sont dans un support pharmaceutique pour l'administration parentérale à un patient.

9. Composition à libération prolongée pour l'érythropoïétine biologiquement active comprenant de l'érythropoïétine incorporée dans le polymère d'une microsphère polymère biodégradable, dans laquelle l'érythropoïétine est dans une concentration située entre 0,01 et 20 % en poids, contenant entre 50 et 40 000 unités/mg et en combinaison avec un agent stabilisant choisi parmi le groupe constitué des acides aminés, des tensioactifs, des sels et des lipides, dans laquelle le rapport de l'agent stabilisant sur la protéine de l'érythropoïétine est situé entre 1 : 10 et 4 : 1 pour les acides aminés sur la protéine et le sel sur la protéine, entre 1 : 1000 et 1 : 20 pour le tensioactif sur la protéine, et entre 1 : 20 et 4 : 1 pour les lipides sur la protéine.

10. Composition de la revendication 9, dans laquelle la microsphère polymère biodégradable est telle que définie dans l'une quelconque des revendications 3 à 6.

11. Composition de la revendication 9 ou 10, dans laquelle les microsphères sont présentes dans un support pharmaceutiquement acceptable.

12. Composition de la revendication 9, comprenant en outre des excipients modulant le taux d'érosion du polymère choisis parmi le groupe constitué des acides inorganiques, des bases inorganiques, des bases organiques, des acides organiques, des sels, des agents complexants, des hydrates de carbone et des tensioactifs.

13. Composition de la revendication 12, dans laquelle l'excipient modulant le taux d'érosion du polymère est un agent porogène tel qu'un sel ou un sucre et est ajouté au polymère sous forme particulaire en une concentration située entre un et trente pour-cent (p/p, polymère).

14. Composition de la revendication 9, comprenant en outre des excipients modifiant la solubilité de l'EPO choisis parmi le groupe constitué des sels, des acides inorganiques, des acides organiques, des bases inorganiques, des bases organiques et des tensioactifs et présents en une concentration située entre 0,1 et trente pour-cent (p/p, polymère).

15. Composition susceptible d'être obtenue par le procédé de l'une quelconque des revendications 1 à 9.

16. Composition à libération prolongée pour l'érythropoïétine biologiquement active, comprenant de l'érythropoïétine incorporée dans le polymère d'une microsphère polymère biodégradable, dans laquelle l'érythropoïétine est présente en une concentration située entre 0,01 et 20 % en poids, du sulfate d'ammonium, un carbonate métallique et un agent stabilisant choisi parmi le groupe constitué des acides aminés, des tensioactifs, des sels et des lipides, dans lequel le rapport de l'agent stabilisant sur la protéine de l'érythropoïétine est situé entre 1 : 10 et 4 : 1 pour les acides aminés sur la protéine et le sel sur la protéine, entre 1 : 1000 et 1 : 20 pour le tensioactif sur la protéine et entre 1 : 20 et 4 : 1 pour les lipides sur la protéine.

## Revendications (Revendications pour l'(les) Etat(s) contractant(s) suivant(s): IE, PT)

1. Procédé de fabrication d'une composition à libération prolongée pour l'érythropoïétine biologiquement active destinée à une administration à un patient, comprenant l'incorporation de l'érythropoïétine dans une microsphère polymère biodégradable de manière à ce que ladite érythropoïétine soit incorporée dans le polymère de ladite microsphère, dans lequel l'érythropoïétine est dans une concentration située entre 0,01 et 20 % en poids, et en combinaison avec un agent stabilisant choisi parmi le groupe constitué des hydrates de carbone des protéines, des acides aminés, des tensioactifs, des sels et des lipides, dans lequel le rapport de l'agent stabilisant sur la protéine de l'érythropoïétine est situé entre 1 : 10 et 4 : 1 pour les hydrates de carbone sur la protéine, les acides aminés sur la protéine et le stabilisant de la protéine sur la protéine et le sel sur la protéine, entre 1 : 1000 et 1 : 20 pour le tensioactif sur la protéine et entre 1 : 20 et 4 : 1 pour les lipides sur la protéine.

2. Procédé de la revendication 1, dans lequel la microsphère est une microsphère de protéine produite en
a) mettant en contact une solution de protéine contenant au moins un type de protéine avec un deuxième liquide, qui est d'une miscibilité limitée avec le solvant de protéine et ne dissout pas la protéine pour former un mélange de protéine et de non solvant ;
b) agitant le mélange de protéine et de non solvant pour former une dispersion de la solution de protéine dans le deuxième liquide ; et
c) éliminant le solvant de protéine pour former une microsphère de protéine,
dans lequel la microsphère de protéine a un diamètre situé entre 50 nm et 100 microns et la microsphère n'est pas formée par des liaisons amide ou une dénaturation thermique de la protéine.

3. Procédé de la revendication 2, dans lequel la protéine est une protéine hydrophobe.

4. Procédé de la revendication 3, dans lequel la protéine hydrophobe est choisie parmi le groupe constitué de la prolamine, du collagène, de la caséine et de la kératine.

5. Procédé de la revendication 1, dans lequel la microsphère est formée d'un polymère synthétique choisi parmi le groupe constitué des poly(lactides), poly(lactides-co-glycolides), poly(caprolactones), polycarbonates, polyamides, polyanhydrides, polyaminoacides, polyortho-esters, polyacétals, polycyanoacrylates, polyuréthannes, polyacrylates, polymères d'éthylène - acétate de vinyle, polystyrènes, poly(chlorures de vinyle), poly(fluorures de vinyle), poly(vinyl imidazoles), polyoléfines chlorosulfonées, poly(oxydes d'éthylène), polyphosphazènes, polyhydroxybutyrates, de leurs mélanges et copolymères.

6. Procédé de la revendication 1, dans lequel la microsphère est formée d'un polymère non protéine en combinaison avec une protéine.

7. Procédé de la revendication 1, dans lequel les microsphères sont dans un support pharmaceutique pour l'administration entérale à un patient.

8. Procédé de la revendication 1, dans lequel les microsphères sont dans un support pharmaceutique pour l'administration parentérale à un patient.

9. Composition à libération prolongée pour l'érythropoïétine biologiquement active comprenant de l'érythropoïétine incorporée dans le polymère d'une microsphère polymère biodégradable, dans laquelle l'érythropoïétine est dans une concentration située entre 0,01 et 20 % en poids, contenant entre 50 et 40 000 unités/mg et en combinaison avec un agent stabilisant choisi parmi le groupe constitué des hydrates de carbone, des protéines, des acides aminés, des tensioactifs, des sels et des lipides, dans laquelle le rapport de l'agent stabilisant sur la protéine de l'érythropoïétine est situé entre 1 : 10 et 4 : 1 pour les hydrates de carbone sur la protéine, les acides aminés sur la protéine, le stabilisant de la protéine sur la protéine, et le sel sur la protéine, entre 1 : 1000 et 1 : 20 pour le tensioactif sur la protéine et entre 1 : 20 et 4 : 1 pour les lipides sur la protéine.

10. Composition de la revendication 9, dans laquelle la microsphère polymère biodégradable est telle que définie dans l'une quelconque des revendications 3 à 6.

11. Composition des revendications 9 ou 10, dans laquelle les microsphères sont présentes dans un support pharmaceutiquement acceptable.
